# EUROPEAN PATENT SPECIFICATION

(11) **EP 1 307 175 B1**
(45) Date of publication and mention of the grant of the patent: **15.02.2006**
(21) Application number: 01960615.1
(22) Date of filing: 02.08.2001
(51) Int. Cl.: A61K 8/04, A61K 8/891, A61K 8/896, A61K 8/91, A61Q 5/06

(54) **HAIR STYLING COMPOSITION**
HAARFORMUNGSZUSAMMENSETZUNG
COMPOSITION DE MISE EN FORME DES CHEVEUX

(30) Priority: 08.08.2000 GB 0019493
(43) Date of publication of application: 07.05.2003
(73) Proprietor: UNILEVER PLC, London, Greater London EC4P 4BQ (GB); UNILEVER N.V., 3013 AL Rotterdam (NL)
(72) Inventor: KHOSHDEL, Ezat, Unilever Research Port Sunlight, Wirral, Merseyside CH63 3J W (GB); PRATLEY, Stuart Keith, Unilever Res Port Sunlight, Wirral, Merseyside CH63 3JW (GB); RUTHERFORD, Keith L, Unilever Home&Pers. Care USA, Rolling Meadows, IL 60008 (US)
(74) Representative: Tansley, Sally Elizabeth
(86) International application number: PCT/EP2001/008952
(87) International publication number: WO 2002/011686

(56) References cited:
- WO-A-00/07551
- WO-A-95/06079
- US-A- 4 711 932

## Description

### Field of the Invention

The present invention relates to hair styling compositions, for example creams, gels and especially aerosol hair styling mousse compositions, which deliver excellent styling as well as sensory feel.

### Background and Prior Art

Style creation products such as hair styling mousses provide human hair with a temporary set which can be removed by water or by shampooing, and function by applying a thin film of a resin or gum onto the hair to adhere adjacent hairs together so that they retain the particular shape or configuration at the time of application.

EP 818 190 describes how an emulsion polymerised silicone material having a particular, defined level of cross-linking, and which is cross-linked in emulsion form can be incorporated into a hair styling composition, such as a mousse, gel or cream, to give a formulation which delivers excellent style creation and longevity, whilst leaving the hair soft and natural. An important feature of these systems is the phase behaviour of the silicone, which is said to form a separate high viscosity aggregated phase in the composition. This phase behaviour is considered to be key to effective style creation.

A problem with "aggregating" systems as described in EP 818 190 is that they can tend to gel and form lumps under conditions of prolonged, high temperature storage.

International patent application no PCT/EP99/07427 discloses the use of certain non-ionic surfactants of specified HLB value for use in compositions comprising silicone materials of the type described in EP 818 190. The resulting systems do not form a separate high viscosity aggregated phase and yet nevertheless deliver effective style creation.

Graft copolymers of polyvinyl alcohol on polyalkylene glycols are known. For example, DE 1 077 430 describes a process for the preparation of graft polymers of vinyl esters on polyalkylene glycols. The preparation of graft copolymers of polyvinyl alcohol on polyalkylene glycols by hydrolysis of the vinyl esters is described in DE 1 094 457 and DE 1 081 229. The use of water-soluble or water-dispersible polymerisates, obtained by the polymerisation of a vinyl ester in the presence of a polyether, as coating agents, binding agents or film-forming auxiliary agents in pharmaceutical formulations, is described in WO 00/18375. The use of the polymers in formulations of hair cosmetics is described in WO 00/49998, published on 31 August 2000.

The use of cross-linked silicones in hair styling compositions improves the feel of hair whilst maintaining hold. WO 00/07551 describes hair treatment compositions containing silicone, preferably cross-linked, and hydrocarbon materials, for improving fullness, body and volume to the hair. There still exists, however, a need for hair styling compositions having yet further improved properties.

The present invention is based on the surprising finding that hair styling compositions containing a copolymer of polyvinyl alcohol and polyalkylene glycols, such as are obtainable by graft polymerisation, in addition to a cross-linked silicone, have improved feel and/or hold compared to compositions which do not contain the copolymer.

### SUMMARY OF THE INVENTION

The present invention provides a hair styling composition comprising:
(i) from 0.1% to 10% by weight, based on total weight, of a non-rigid emulsion polymerised cross-linked silicone polymer, in which the percentage of branched monomer units in the silicone polymer is from 0.05% to 10%;
(ii) from 0.01% to 10% by weight, based on total weight, of a copolymer having a polyethylene backbone comprising a polyether and, depending from said polyethyleneglycol backbone, a plurality of poly (vinyl ester) groups, wherein at least 95% ester groups are hydrolysed to the corresponding alcohol; and wherein the polymer has a molar ratio of polyether to total poly (vinyl ester) and polyvinyl alcohol groups from 30:70 to 50:50; and

water.

In another aspect, the invention provides a method of styling hair which comprises applying to the hair a composition of the invention.

A yet further aspect of the invention is the use of the compositions of the invention for the styling of hair.

### DETAILED DESCRIPTION

### Cross-linked Silicone Polymer

The non-rigid emulsion-polymerised cross-linked silicone polymer (i) is present in compositions of the invention in an amount from 0.1% to 10% by weight based on the total weight of the composition, more preferably from 0.2% to 6% by weight, most preferably from 0.5 to 5% by weight.

Preferred silicone polymers for use in the invention are polydiorganosiloxanes, preferably derived from suitable combinations of R₃SiO_{0.5} units and R₂SiO units where each R independently represents an alkyl, alkenyl (e.g., vinyl), alkaryl, aralkyl, or aryl (e.g. phenyl) group. R is most preferably methyl.

The preferred silicone polymers of the invention are cross-linked polydimethyl siloxanes (which have the CTFA designation dimethicone), and cross-linked polydimethyl siloxanes having end groups such as hydroxyl (which have the CTFA designation dimethiconol). Good results have been obtained with cross-linked dimethiconol.

Cross-linking of the silicone polymer is typically introduced concurrently during emulsion polymerisation of the polymer through the inclusion of the required amount of trifunctional and tetrafunctional silane monomer units, for example, those of formula:
R Si (OH)₃ wherein R represents an alkyl, alkenyl (e.g. vinyl), alkaryl, aralkyl or aryl (e.g. phenyl) group, preferably methyl.

The degree of cross-linking of the silicone polymer can be measured as the percentage of branched monomer units in the silicone polymer and is from 0.05% to 10%, preferably being in the range 0.15% to 7%, e.g. from 0.2% to 2%. It will be understood by those skilled in the art that the percentage of branched monomer units in the silicone polymer is a percentage by weight. Increasing cross-linking is found to improve styling benefits but also to reduce conditioning performance somewhat, so compromise levels must be selected with properties optimised to suit consumer preferences in different cases. Good overall performance has been obtained with dimethiconol 0.3% cross-linked.

It is highly preferred if the polymer i) is.a cross-linked dimethiconal, having a percentage of branched monomer units in the silicone polymer in the range 0.15 to 7%.

Suitable emulsion polymerised cross-linked silicone polymers are commercially available or can be readily made using conventional techniques well known to those skilled in the art.

Croes-linked silicone polymers are described in EP 818190, the contents of which are incorporated herein by reference.

### Copolymer Having A Polyether Backbone

The compositions of the invention comprise from 0.01% to 10% weight of a copolymer having a backbone (or main chain; the terms backbone and main chain are used synonymously herein) comprising a polyether and, depending from the polyethylene glycol backbone, a plurality of poly (vinyl ester) groups. At least 95% the ester groups are hydrolysed to the corresponding alcohol. The poly (vinyl eater) chains optionally contain other functional groups in and/or on the polymer chain, such as, for example, amide and/or keto groups.

The copolymer comprises a polyethyleneglycol backbone.

The copolymer is preferably polyethyleneglycol-co-polyvinylalcohol having polyvinylalcohol groups bound to the polyethyleneglycol i.e., substantially all of the poly (vinyl ester) groups are hydrolysed in the copolymers used in the compositions of the invention.

The copolymer can be produced by methods which are well-known to those skilled in the art. For example, the copolymers are obtainable by graft polymerisation. In a method comprising graft polymerisation, poly (vinyl ester) groups are preferably grafted onto a polyether and are subsequently hydrolysed to convert at least some of the ester groups to the corresponding alcohol. For example, DE 1 077 430 describes a process for the preparation of graft polymers of vinyl esters on polyalkylene glycols. The preparation of graft copolymers of polyvinyl alcohol on polyalkylene glycols by hydrolysis of the vinyl esters is described in DE 1 094 457 and DE 1 081 229.

The weight average molecular weight of the polyether is preferably from 1 to 100 kDa.

Copolymers for use in compositions of the invention have a molar ratio of polyether to total poly(vinyl ester) and polyvinylalcohol groups in the range of from 30:70 to 50:50. Typically, such copolymers have a molar ratio of polyether to total poly(vinyl ester) and polyvinylalcohol groups of about 40:60.

The copolymer may be non-cross-linked or cross-linked and it is preferred that the copolymer is cross-linked. Suitable cross-linking agents are those compounds which can bind to two or more polyether, poly (vinyl ester) and/or poly (vinyl alcohol) chains and include, for example, pentaerythritol triallyl ether.

The copolymer which is used in the invention, and the methods for its preparation, are further described below.

Broadly, the copolymer which may be used in the invention is obtainable by free radical polymerisation of:
a) at least one vinyl ester; in the presence of
b) polyether-containing compounds; and
c) optionally at least one other copolymerisable monomer;

followed by subsequent at least partial hydrolysis of the ester functions of the original monomers (a).

In the preparation of the polymers used according to the invention, grafting onto the polyether-containing compounds (b) can result during the polymerization, which can lead to advantageous properties for the polymers. However, mechanisms other than grafting are also possible.

Depending on the degree of grafting, the polymers used according to the invention are taken to mean pure graft polymers and also mixtures of the abovementioned graft polymers with non-grafted polyether-containing compounds and homo- or copolymers of the monomers (a) and (c).

Polyether-containing compounds (b) which can be used are either based on ethylene oxide, or polyglycerol.

The structural units can either be homopolymers or random copolymers and block copolymers.

The preferred ethylenically unsaturated comonomer (c) can be described by the following formula:

X -C (O)CR¹⁵=CHR¹⁴

where
X is chosen from the group of radicals -OH, -OM, -OR¹⁶, NH₂, NHR¹⁶, N(R¹⁶)₂;
M is a cation chosen from the group consisting of: Na⁺, K⁺, Mg⁺⁺, Ca⁺⁺, Zn⁺⁺, NH₄⁺, alkyl ammonium, dialkylammonium, trialkylammonium and tetraalkylammonium.

The R¹⁶ radicals can be identical or different and are chosen from the group consisting of: -H, C₁-C₄₀ linear or branched alkyl radicals, N, N-dimethylaminoethyl, 2-hydroxyethyl, 2-methoxyethyl, 2-ethoxyethyl, hydroxypropyl, methoxypropyl or ethoxypropyl.

R¹⁵ and R¹⁴ are chosen, independently, from the group consisting of: -H, C₁-C₈ linear or branched alkyl chains, methoxy, ethoxy, 2-hydroxyethoxy, 2-methoxyethoxy and 2-ethoxyethyl.

Representative, but non-limiting, examples of suitable (c) monomers are: acrylic or methacrylic acid and salts, and esters and amides thereof. The salts can be derived from any desired non-toxic metal, ammonium or substituted ammonium counter-ions.

The esters can be derived from: C₁-C₄₀ linear, C₃-C₄₀ branched or C₃-C₄₀ carbocyclic alcohols, from polyfunctional alcohols, having from 2 to about 8 hydroxyl groups, such as ethylene glycol, hexylene glycol, glycerol and 1, 2, 6-hexanetriol, from aminoalcohols or alcohol ethers such as methoxyethanol and ethoxyethanol, (alkyl)polyethylene glycols, (alkyl)polypropylene glycols or ethoxylated fatty alcohols, for example C₁₂-C₂₄ fatty alcohols reacted with 1 to 200 ethylene oxide units.

Also suitable are N,N-dialkylaminoalkyl acrylates and methacrylates and N-dialkylaminoalkylacryl- and -methacrylamides of general formula (III). 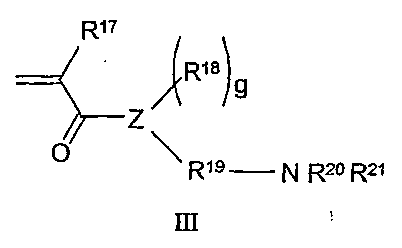
where
- R¹⁷ =: H, alkyl having from 1 to 8 carbon atoms,
- R¹⁸ =: H, methyl,
- R¹⁹ =: alkylene having from 1 to 24 carbon atoms, optionally substituted by alkyl,
- R²⁰, R²¹ =: C₁ - C₄₀ alkyl radical,
- Z =: nitrogen when g = 1, or oxygen when g = 0.

The amides can be unsubstituted, N-alkyl- or N-alkylamino-mono-substituted or N,N-dialkyl-substituted or N,N-dialkylamino- di-substituted, where the alkyl or alkylamino groups are derived from C₁-C₄₀ linear, C₃-C₄₀ branched, or C₃ -C₄₀ carbocyclic units. In addition, the alkylamino groups can be quaternized.

Preferred examples of comonomers described by formula III are N,N-dimethylaminomethyl (meth)acrylate, N,N-diethylaminomethyl (meth)acrylate, N,N-dimethylaminoethyl (meth)acrylate, N,N-diethylaminoethyl (meth)acrylate, N-[3-(dimethylamino)propyl]methacrylamide and N-[3-(dimethylamino)propyl]acrylamide.

Monomers which can likewise be used as comonomer (c) are: substituted acrylic acids and salts, esters and amides thereof, where the substituents on the carbon atoms are in the two or three position of the acrylic acid, and are chosen, independently, from the group consisting of C₁-C₄ alkyl, -CN, and COOH. Methacrylic acid, ethacrylic acid and 3-cyanoacrylic acid are particularly preferred. Salts, esters and amides of these substituted acrylic acids can be chosen as described above for the salts, esters and amides of acrylic acid.

Other suitable group (c) monomers are: allyl esters of C₁-C₄₀ linear, C₃-C₄₀ branched or C₃ - C₄₀ carbocyclic carboxylic acids, vinyl or allyl halides, preferably vinyl chloride and allyl chloride, vinyl ethers, preferably methyl, ethyl, butyl or dodecyl vinyl ether, vinylformamide, vinylmethylacetamide, vinylamine; vinyllactams, preferably vinylpyrrolidone and vinylcaprolactam, vinyl- or allyl-substituted heterocyclic compounds, preferably vinylpyridine, vinyloxazoline and allylpyridine.

Also suitable are N-vinylimidazoles of general formula IV, in which R²² to R²⁴ are chosen, independently, from hydrogen, C₁-C₄ - alkyl or phenyl.

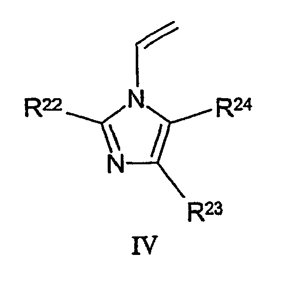

Other suitable (c) comonomers are diallylamines with general formula (V) 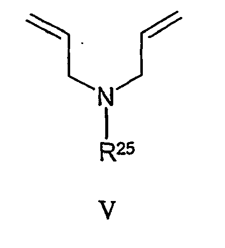
where R²⁵ = C₁-C₂₄ alkyl.

Other suitable (c) comonomers are vinylidene chloride and hydrocarbons having at least one carbon-carbon double bond, preferably chosen from: styrene, alpha-methylstyrene, tert-butylstyrene, butadiene, isoprene, cyclohexadiene, ethylene, propylene, 1-butene, 2-butene, isobutylene, vinyltoluene, and mixtures of these monomers.

Particularly suitable (c) comonomers are: acrylic acid, methacrylic acid, ethyl acrylic acid, methyl acrylate, ethyl acrylate, propyl acrylate, n-butyl acrylate, iso-butyl acrylate, t-butyl acrylate, 2-ethylhexyl acrylate, decyl acrylate, methyl methacrylate, ethyl methacrylate, propyl methacrylate, n-butyl methacrylate, iso-butyl methacrylate, t-butyl methacrylate, 2-ethylhexyl methacrylate, decyl methacrylate, methyl ethacrylate, ethyl ethacrylate, n-butyl ethacrylate, iso-butyl ethacrylate, t-butyl ethacrylate, 2-ethylhexyl ethacrylate, decyl ethacrylate, stearyl (meth)acrylate, 2,3-dihydroxypropyl acrylate, 2,3-dihydroxypropyl methacrylate, 2-hydroxyethyl acrylate, hydroxypropyl acrylates, 2-hydroxyethyl methacrylate, 2-hydroxyethyl ethacrylate, 2-methoxyethyl acrylate, 2-methoxyethyl methacrylate, 2-methoxyethyl ethacrylate, 2-ethoxyethyl methacrylate, 2-ethoxyethyl ethacrylate, hydroxypropyl methacrylates, glyceryl monoacrylate, glyceryl monomethacrylate, polyalkylene glycol (meth)acrylates, unsaturated sulfonic acids such as, for example, acrylamidopropane sulfonic acid;
acrylamide, methacrylamide, ethacrylamide, N-methylacrylamide, N,N-dimethylacrylamide, N-ethylacrylamide, N-isopropylacrylamide, N-butylacrylamide, N-t-butylacrylamide, N-octylacrylamide, N-t-octylacrylamide, N-octadecylacrylamide, N-phenylacrylamide, N-methylmethacrylamide, N-ethylmethacrylamide, N-dodecylmethacrylamide, 1-vinylimidazole, 1-vinyl-2-methylvinylimidazole, N,N-dimethylaminomethyl (meth)acrylate, N,N-diethylaminomethyl (meth)acrylate, N,N-dimethylaminoethyl (meth)acrylate, N,N-diethylaminoethyl (meth)acrylate, N,N-dimethylaminobutyl (meth)acrylate, N,N-diethylaminobutyl (meth)acrylate, N,N-dimethylaminohexyl (meth)acrylate, N,N-dimethylaminooctyl (meth)acrylate, N,N-dimethylaminododecyl (meth)acrylate, N-[3-(dimethylamino)propyl]methacrylamide, N-[3-(dimethylamino)propyl]acrylamide, N-[3(-dimethylamino)butyl]methacrylamide, N-[8-(dimethylamino)octyl]methacrylamide, N-[12-(dimethylamino)dodecyl]methacrylamide, N-[3-(diethylamino)propyl]methacryamide, N-[3-(diethylamino)propyl]acrylamide;
maleic acid, fumaric acid, maleic anhydride and its half-esters, crotonic acid, itaconic acid, diallyldimethylammonium chloride, vinyl ethers (for example: methyl, ethyl, butyl or dodecyl vinyl ether), vinyl formamide, vinylmethylacetamide, vinylamine; methyl vinyl ketone, maleimide, vinylpyridine, vinylimidazole, vinylfuran, styrene, styrene sulfonate, allyl alcohol, and mixtures thereof.

Of these, particular preference is given to: acrylic acid, methacrylic acid, maleic acid, fumaric acid, crotonic acid, maleic anhydride and its half-esters, methyl acrylate, methyl methacrylate, ethyl acrylate, ethyl methacrylate, n-butyl acrylate, n-butyl methacrylate, t-butyl acrylate, t-butyl methacrylate, iso-butyl acrylate, iso-butyl methacrylate, 2-ethylhexyl acrylate, stearyl acrylate, stearyl methacrylate, N-t-butylacrylamide, N-octylacrylamide, 2-hydroxyethyl acrylate, hydroxypropyl acrylate, 2-hydroxyethyl methacrylate, hydroxypropyl methacrylate, alkylene glycol (meth)acrylates, styrene, unsaturated sulfonic acids such as, for example, acrylamidopropane sulfonic acid, vinylpyrrolidone, vinylcaprolactam, vinyl ethers (e.g.: methyl, ethyl, butyl or dodecyl vinyl ether), vinylformamide, vinylmethylacetamide, vinylamine, 1-vinylimidazole, 1-vinyl-2-methylimidazole, N,N-dimethylaminomethyl methacrylate and N-[3-(dimethylamino)propyl]methacrylamide; 3-methyl-1-vinylimidazolium chloride, 3-methyl-1-vinylimidazolium methylsulfate, N,N-dimethylaminoethyl methacrylate, N-[3-(dimethylamino)propyl]methacrylamide quaternized with methyl chloride, methyl sulfate or diethyl sulfate.

Monomers having one basic nitrogen atom can be quaternized with: alkyl halides having from 1 to 24 carbon atoms in the alkyl group, e.g. methyl chloride, methyl bromide, methyl iodide, ethyl chloride, ethyl bromide, propyl chloride, hexyl chloride, dodecyl chloride, lauryl chloride and benzyl halides, in particular benzyl chloride and benzyl bromide. Other suitable quaternizing agents are dialkyl sulfates, in particular dimethyl sulfate or diethyl sulfate. The quaternization of the basic amines can also be carried out with alkylene oxides such as ethylene oxide or propylene oxide in the presence of acids. Preferred quaternizing agents are: methyl chloride, dimethyl sulfate or diethyl sulfate.

The quaternization can, in addition, be carried out before or after polymerization.

Furthermore, it is possible to use the product derived from reacting unsaturated acids, for example, acrylic or methacrylic acid, with a quaternized epichlorohydrin of general formula (VI) (R²⁶ = C₁ to C₄₀ -alkyl).

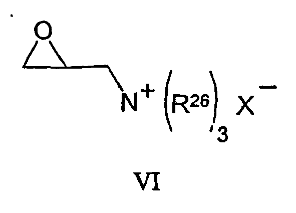

Examples thereof are:
(meth)acryloyloxyhydroxypropyltrimethylammonium chloride and
(meth)acryloyloxyhydroxypropyltriethylammonium chloride.

The basic monomers can also be cationized, by neutralizing them with mineral acids, for example, sulfuric acid, hydrochloric acid, hydrobromic acid, hydroiodic acid, phosphoric acid or nitric acid, or with organic acids, for example, formic acid, acetic acid, lactic acid, or citric acid.

In addition to the abovementioned comonomers, it is also possible to select as comonomer (c), so-called macromonomers, such as, for example, silicone-containing macromonomers having one or more free-radically polymerizable groups or alkyloxazoline macromonomers, as described, for example, in EP 408 311.

Furthermore, it is possible to use fluorine-containing monomers, as described, for example, in EP 558423 and cross-linking compounds or compounds which regulate the molecular weight, in combination or alone.

Regulators which can be used are the customary compounds known to the person skilled in the art, such as, for example, sulfur compounds (e.g. mercaptoethanol, 2-ethylhexyl thioglycolate, thioglycolic acid or dodecylmercaptan), and tribromochloromethane and other compounds which have a regulating effect on the molecular weight of the resulting polymers.

In some instances, it is also possible to use thio-containing silicone compounds. Preference is given, however, to using silicone-free regulators.

Suitable cross-linking monomers are compounds which possess at least two ethylenically unsaturated double bonds, and include, for example, esters derived from ethylenically unsaturated carboxylic acids, such as acrylic or methacrylic acid, and polyhydric alcohols, ethers of at least dihydric alcohols such as, for example, vinyl ethers or allyl ethers. Also suitable are straight-chain or branched, linear or cyclic aliphatic or aromatic hydrocarbons possessing at least two double bonds which, in the case of the aliphatic hydrocarbons, must not be conjugated. Also suitable are amides of acrylic and methacrylic acid and N-allylamines of at least difunctional amines, for example, 1,2-diaminoethane and 1,3-diaminopropane. Also suitable are triallylamine or corresponding ammonium salts, N-vinyl compounds of urea derivatives, at least difunctional amides, cyanurates or urethanes. Other suitable cross-linkers are divinyldioxane, tetraallylsilane or tetravinylsilane.

Particularly preferred cross-linkers are, for example, methylenebisacrylamide, triallylamine and triallylammonium salts, divinylimidazole, N,N'-divinylethyleneurea, reaction products of polyhydric alcohols with acrylic, or methacrylic acid, methacrylic esters and acrylic esters of polyalkylene oxides or polyhydric alcohols which have been reacted with ethylene oxide and/or propylene oxide and/or epichlorohydrin.

It is also possible, in some instances, for other polymers, such as, for example, polyamides, polyurethanes, polyesters, homo- and copolymers of ethylenically unsaturated monomers, to be present during the polymerisation for preparation of the polymers according to the invention. Examples of such polymers, some of which are also used in cosmetics, are the polymers known under the tradenames Amerhold™, Ultrahold™, Ultrahold Strong™, LuvifleX™, VBM, Luvimer™, Acronal™, Acudyne™, Stepanhold™, Lovocryl™, Versatyl™, Amphomer™ or Eastma AQ™.

The (c) comonomers according to the invention can, provided they contain ionizable groups, be partially or completely neutralized using acids or bases before or after the polymerization in order, for example, to adjust the solubility or dispersibility in water to the desired degree.

Examples of suitable neutralizing agents for monomers carrying acidic groups are: mineral bases such as sodium carbonate, alkali metal hydroxides and ammonia; organic bases such as aminoalcohols, specifically 2-amino-2-methyl-1-propanol, monoethanolamine, diethanolamine, triethanolamine, triisopropanolamine, tri[(2-hydroxy)-1-propyl]amine, 2-amino-2-methyl-1,3-propanediol, 2-amino-2-hydroxymethyl-1,3-propanediol and diamines, such as, for example, lysine.

To prepare the polymers, the monomers of component a) can be polymerized in the presence of the polyethers using initiators which form free radicals, or by the action of high-energy radiation, which is also intended to mean the action of high-energy electrons.

Initiators which can be used for free-radical polymerization are the customary peroxo and/or azo compounds used for this purpose, and include, for example: alkali metal or ammonium peroxydisulfates, diacetyl peroxide, dibenzoyl peroxide, succinyl peroxide, di-tert-butyl peroxide, tert-butyl perbenzoate, tert-butyl perpivalate, tert-butyl permaleate, cumene hydroperoxide, di-isopropyl peroxydicarbamate, bis-(o-toluoyl) peroxide, didecanoyl peroxide, dioctanoyl peroxide, dilauroyl peroxide, tert-butyl perisobutyrate, tert-butyl peracetate, di-tert-amyl peroxide, tert-butyl hydroperoxide, azobisisobutyronitrile, azobis-(2-amidinopropane) dihydrochloride or 2,2'-azobis(2-methylbutyronitrile). Also suitable are initiator mixtures or redox initiator systems, such as, for example, ascorbic acid/iron(II) sulfate/sodium peroxodisulfate, tert-butyl hydroperoxide/sodium disulfite, tert-butyl hydroperoxide/sodium hydroxymethanesulfinate.

Preference is given to using organic peroxides.

The amounts of initiator or initiator mixtures used, based on monomer used, are between 0.01 and 10% by weight, preferably between 0.1 and 5% by weight.

The polymerization is carried out in a temperature range from 40 to 200°C, preferably in the range from 50 to 140°C, particularly preferably in the range from 60 to 110°C. It is usually carried out under atmospheric pressure, but can also be carried out under reduced or increased pressure, preferably between 1 and 5 bar.

The polymerization can, for example, be carried out as solution polymerization, bulk polymerization, emulsion polymerization, inverse emulsion polymerization, suspension polymerization, inverse suspension polymerization or precipitation polymerization, without the possible methods being limited thereto.

In the case of bulk polymerization, the procedure may involve dissolving the polyether-containing compound b) in at least one monomer of group a) and possibly other comonomers of group c) and, after the addition of a polymerization initiator, fully polymerizing the mixture. The polymerization can also be carried out semi-continuously by firstly introducing some, e.g. 10%, of the mixture to be polymerized, comprising the polyether-containing compound b), at least one monomer from group a), possibly other comonomers of group c) and initiator, heating the mixture to the polymerization temperature and after the polymerization has started, adding the remainder of the mixture to be polymerized in accordance with the progress of the polymerization. The polymers can also be obtained by initially introducing the polyether-containing compounds of group b) into a reactor, heating them to the polymerization temperature and adding at least one monomer of group a), possibly other comonomers of group c) and polymerization initiator either in one portion, step by step or, preferably, continuously, and polymerizing.

If desired, the above described polymerization can also be carried out in a solvent. Suitable solvents are, for example, alcohols, such as methanol, ethanol, n-propanol, iso-propanol, n-butanol, sec-butanol, tert-butanol, n-hexanol and cyclohexanol, and glycols, such as ethylene glycol, propylene glycol and butylene glycol, and the methyl or ethyl ethers of dihydric alchols, diethylene glycol, triethylene glycol, glycerol and dioxane. The polymerization can also be carried out in water as solvent. In this case, the initial batch is a solution which, depending on the amount of monomers of component a) added, is soluble in water to a greater or lesser degree. In order to convert water-insoluble products, which can form during the polymerization, into solution, it is possible, for example, to add organic solvents, such as monohydric alcohols having from 1 to 3 carbon atoms, acetone or dimethylformamide. However, in the case of polymerization in water, it is also possible to convert the water-insoluble polymers into a finely divided dispersion by addition of customary emulsifiers or protective colloids, e.g. polyvinyl alcohol.

The emulsifiers used are, for example, ionic or non-ionic surfactants whose HLB value is in the range from 3 to 13. The definition of the HLB value can be found in the publication by W.C. Griffin, J. Soc. Cosmetic Chem., Volume 5, 249 (1954).

The amount of surfactants, based on the polymer, is from 0.1 to 10% by weight. Using water as solvent gives solutions or dispersions of the polymers. If solutions of the polymer are prepared in an organic solvent, or in mixtures of an organic solvent and water, then, per 100 parts by weight of the polymer, from 5 to 2000, preferably from 10 to 500, parts by weight of the organic solvent or of the solvent mixture are used.

Preference is given to polymers obtainable by free-radical polymerization of
a) 10 - 98 % by weight of at least one vinyl ester of C₁ - C₂₄ carboxylic acids in the presence of
b) 2 - 90 % by weight of at least one polyether-containing compound and
c) 0 - 50 % by weight of one or more other copolymerizable monomers.

Particular preference is given to polymers obtainable by free-radical polymerization of
a) 50 - 97 % by weight of at least one vinyl ester of C₁ - C₂₄ carboxylic acids in the presence of
b) 3 - 50 % by weight of at least one polyether-containing compound and
c) 0 - 30 % by weight of one or more other copolymerizable monomers.

Very particular preference is given to polymers obtainable by free-radical polymerization of
a) 65 - 97 % by weight of at least one vinyl ester of C₁ - C₂₄ carboxylic acids in the presence of
b) 3 - 40 % by weight of at least one polyether-containing compound and
c) 0 - 20 % by weight of one or more other copolymerizable monomers.

To prepare the polymers used according to the invention, the ester groups of the original monomers a) and optionally of other monomers are cleaved after the polymerization by hydrolysis, alcoholysis, or aminolysis. This process step is generally referred to below as hydrolysis. The hydrolysis takes place in a manner known *per se* by the addition of a base, preferably the addition of a sodium or potassium hydroxide solution in water and/or alcohol. Particular preference is given to using methanolic sodium or potassium hydroxide solutions. The hydrolysis is carried out at temperatures in the range from 10 to 80°C, preferably in the range from 20 to 60°C. The degree of hydrolysis depends on the amount of base used, on the hydrolysis temperature, on the hydrolysis time and the water content of the solution.

After the hydrolysis, the polymer solutions can be steam distilled to remove any solvents. After the steam distillation, aqueous solutions or dispersions are obtained depending on the degree of hydrolysis, type of (b) polyethers, of group (a) vinyl esters and any group (c) monomers used.

The polymer solutions and dispersions can be converted into powder form by a variety of drying methods, such as, for example, spray drying, fluidized spray drying, drum drying or freeze drying. The drying method used in preference is spray drying. The dry polymer powder obtained in this way can be used to prepare an aqueous solution or dispersion again, by dissolution or redispersion in water. Conversion into powder form has the advantage of improved storability, easier transportation, and a lower propensity for microbial attack.

Instead of the steam distilled polymer solutions, the alcoholic polymer solutions can be directly converted into powder form.

### Hair Styling Polymer

The compositions of the invention preferably comprise, in addition to the copolymer, a hair styling polymer conventionally used in hair styling compositions. The hair styling polymer which is employed in compositions of the present invention should be capable of forming a film and holding the hair of the user in place.

Preferably the hair styling polymer is present at levels up to 10% by weight, based on total weight of a hair styling polymer.

Hair styling polymers are well known articles of commerce and many such polymers are available commercially which contain functional groups which render the polymers cationic, anionic, amphoteric or non-ionic in character.

Examples of cationic hair styling polymers are copolymers of amino-functionalised acrylate monomers such as lower alkylaminoalkyl acrylate or methacrylate monomers such as dimethylaminoethyl methacrylate with compatible monomers such as N-vinylpyrrolidone, vinyl caprolactam, or alkyl methacrylates such as methyl methacrylate and ethyl methacrylate and alkyl acrylates such as ethyl acrylate and n-butyl acrylate. Cationic hair styling polymers containing N-vinylpyrrolidone are commercially available from ISP Corporation such as those sold under the trademarks of Gafquat® 734, 755 and 755N (quaternary ammonium polymers formed by the reaction of diethyl sulfate and a copolymer of N-vinylpyrrolidone and dimethylaminoethyl methacrylate and having the CTFA designation Polyquaternium-11).

Examples of anionic hair styling polymers are the copolymers of vinyl acetate and crotonic acid, terpolymers of vinyl acetate, crotonic acid and a vinyl ester of an alpha-branched saturated aliphatic monocarboxylic acid such as vinyl neodecanoate; copolymers of methyl vinyl ether and maleic anhydride (molar ratio about 1:1) wherein such copolymers are 50% esterified with a saturated alcohol containing from 1 to 4 carbon atoms such as ethanol or butanol; and acrylic copolymers, terpolymers, etc., containing acrylic acid or methacrylic acid as the anionic radical-containing moiety and esters of acrylic or methacrylic acid with one or more saturated alcohols having from 1 to 22 carbon atoms such as methyl methacrylate, methyl acrylate, ethyl acrylate, ethyl methacrylate, n-butyl acrylate, t-butyl acrylate, t-butyl methacrylate, n-butyl methacrylate, n-hexyl acrylate, n-octyl acrylate, lauryl methacrylate and behenyl acrylate, glycols having from 1 to 6 carbon atoms such as hydroxypropyl methacrylate and hydroxyethyl acrylate, styrene, vinyl caprolactam, vinyl acetate, acrylamide, alkyl acrylamides and methacrylamides having 1 to 8 carbon atoms in the alkyl group such as methacrylamide, t-butyl acrylamide and n-octyl acrylamide, and other compatible unsaturated monomers. One specific example is the emulsion polymerised terpolymer of methacrylic acid, n-butyl acrylate and ethyl acrylate (e.g., in a weight percent ratio of 31:42:27, respectively). Another specific example is the Gantrez® ES series commercially available from ISP corporation (esterified copolymers of methyl vinyl ether and maleic anhydride).

Examples of amphoteric hair styling polymers are those which contain cationic groups derived from monomers such as t-butyl aminoethyl methacrylate as well as carboxyl groups derived from monomers such as acrylic acid or methacrylic acid. One specific example of an amphoteric hair styling polymer is Amphomer® sold by the National Starch and Chemical Corporation.

Examples of non-ionic hair styling polymers are homopolymers of N-vinylpyrrolidone and copolymers of N-vinylpyrrolidone with compatible non-ionic monomers such as vinyl acetate and terpolymers of ethyl acrylate, butyl methacrylate and methyl methacrylate. Non-ionic polymers containing N-vinylpyrrolidone in various weight average molecular weights are available commercially from ISP Corporation such as those sold under the trade marks Copolymer 845 and Copolymer 937 (copolymers of N-vinylpyrrolidone and t-butylaminoethyl methacrylate of average molecular weight about 1,000,000) and homopolymers of N-vinylpyrrolidone having an average molecular weight of about 630,000 sold by ISP Corporation under the tradename PVP K-90 and those having an average molecular weight of about 1,000,000 sold under the tradename PVP K-120.

The hair styling polymers in compositions of the invention are most preferably selected from one or more ionic-type, i.e. cationic and/or anionic, hair styling polymers. Hair styling polymers selected from amphoteric and/or non-ionic hair styling polymers may suitably be used in conjunction with these ionic-type hair styling polymers, to improve, for example, hair styling benefit.

Particularly preferred hair styling polymers in compositions of the invention are those ionic-type hair styling polymers selected from Polyquaternium-16, Polyquaternium 11, Polyquaternium 46 and esterified copolymers of methyl vinyl ether and maleic anhydride, optionally in combination with one or more non-ionic hair styling polymers. Such non-ionic hair styling polymers are preferably selected from vinylpyrrolidone homopolymers and especially copolymers of vinylpyrrolidone and vinyl acetate.

### Nonionic Surfactant

In addition to the cross-linked silicone polymer and the hair styling polymer, the hair styling composition of the invention may, optionally, also include a non-ionic surfactant in an amount of up to 5%, preferably from 0.01% to 1%, most preferably from 0.02% to 0.8% by weight based on total weight.

The HLB (hydrophilic-lipophilic balance) is an important property of the non-ionic surfactant. The property per se and how it is calculated is described in *J.Soc.Cosmet.Chem*.,1949,**1**,311. For a given non-ionic surfactant, the HLB value represents the weight per cent of the hydrophilic content of the molecule divided by a factor of five.

Non-ionic surfactants for use in compositions of the invention typically have an HLB (hydrophilic - lipophilic balance) value of at least 14.5. The HLB value preferably ranges from 15 to 19, most preferably from 16 to 18.

Examples of suitable non-ionic surfactants are condensation products of aliphatic (C₈-C₁₈) primary or secondary linear or branched chain alcohols or phenols with alkylene oxides, usually ethylene oxide and generally having at least 15, preferably at least 20, most preferably from 30 to 50 ethylene oxide groups. Other suitable non-ionics include esters of sorbitol, esters of sorbitan anhydrides, esters of propylene glycol, fatty acid esters of polyethylene glycol, fatty acid esters of polypropylene glycol, ethoxylated esters and polyoxyethylene fatty ether phosphates.

Of particular use are those non-ionic surfactants of general formula R(EO)ₓ H, where R represents a straight or branched chain alkyl group having an average carbon chain length of 12-18 carbon atoms and x ranges from 30 to 50. Specific examples include steareth-40, steareth-50, ceteareth-30, ceteareth-40, ceteareth-50 and mixtures thereof. Suitable commercially available examples of these materials include Unicol SA-40 (Universal Preserv-A-Chem), Empilan KM50 (Albright and Wilson), NONION PS-250 (Nippon Oils & Fats), Volpo CS50 (Croda Inc), and Incropol CS-50 (Croda Inc).

### Water

Compositions of the present invention will also include water, preferably distilled or de-ionised, as a solvent or carrier for the polymers and other components. Water will typically be present in amounts ranging from 30% to 98%, preferably from 60% to 95% by weight based on total weight.

Alcohol may optionally be employed as a co-solvent in compositions of the invention as this can enhance the performance of the styling composition. A suitable alcohol is an aliphatic straight or branched chain monohydric alcohol having 2 to about 4 carbon atoms. Isopropanol and especially ethanol are preferred. A suitable level for the alcohol is up to 20%, preferably from 5% to 15%, by weight based on total weight.

### Product Form

Compositions of the invention may suitably be in aerosol form. A particularly preferred product form is an aerosol hair mousse. Aerosol hair mousse compositions are emitted from the aerosol container as a foam which is then typically worked through the hair with fingers or a hair styling tool and either left on the hair or rinsed out.

Aerosol-form compositions of the invention will include an aerosol propellant which serves to expel the other materials from the container, and forms the mousse character in mousse compositions. The aerosol propellant included in styling compositions of the present invention can be any liquefiable gas conventionally used for aerosol containers. Examples of suitable propellants include dimethyl ether and hydrocarbon propellants such as propane, n-butane and iso-butane. The propellants may be used singly or admixed. Water insoluble propellants, especially hydrocarbons, are preferred because they form emulsion droplets on agitation and create suitable mousse foam densities.

The amount of the propellant used is governed by normal factors well known in the aerosol art. For mousses the level of propellant is generally up to 30%, preferably from 2% to 30%, most preferably from 3% to 15% by weight based on total weight of the composition. If a propellant such as dimethyl ether includes a vapour pressure suppressant (e.g. trichloroethane or dichloromethane), for weight percentage calculations, the amount of suppressant is included as part of the propellant.

The method of preparing aerosol hair styling mousse compositions according to the invention follows conventional aerosol filling procedures. The composition ingredients (not including the propellant) are charged into a suitable pressurisable container which is sealed and then charged with the propellant according to conventional techniques.

Compositions of the invention may also take a non-foaming product form, such as a hair styling cream or gel. Such a cream or gel will include a structurant or thickener, typically at a level of from 0.1% to 10%, preferably 0.5% to 3% by weight based on total weight.

Examples of suitable structurants or thickeners are polymeric thickeners such as carboxyvinyl polymers. A carboxyvinyl polymer is an interpolymer of a monomeric mixture comprising a monomeric olefinically unsaturated carboxylic acid, and from about 0.01% to about 10% by weight of the total monomers of a polyether of a polyhydric alcohol. Carboxyvinyl polymers are substantially insoluble in liquid, volatile organic hydrocarbons and are dimensionally stable on exposure to air. Suitably the molecular weight of the carboxyvinyl polymer is at least 750,000, preferably at least 1,250,000, most preferably at least 3,000,000. Preferred carboxyvinyl polymers are copolymers of acrylic acid cross-linked with polyallylsucrose as described in US Patent 2,798,053. These polymers are provided by B.F.Goodrich Company as, for example, CARBOPOL 934, 940, 941 and 980. Other materials that can also be used as structurants or thickeners include those that can impart a gel-like viscosity to the composition, such as water soluble or colloidally water soluble polymers like cellulose ethers (e.g. methylcellulose, hydroxyethylcellulose, hydroxypropylmethylcellulose and carboxymethylcellulose), guar gum, sodium alginate, gum arabic, xanthan gum, polyvinyl alcohol, polyvinyl pyrrolidone,hydroxypropyl guar gum, starch and starch derivatives, and other thickeners, viscosity modifiers, gelling agents, etc. It is also possible to use inorganic thickeners such as bentonite or laponite clays.

The hair styling compositions of the invention can contain a variety of non-essential, optional components suitable for rendering the compositions more aesthetically acceptable or to aid use, including discharge from the container, of the product. Such conventional optional ingredients are well known to those skilled in the art, e.g. preservatives such as benzyl alcohol, methyl paraben, propyl paraben and imidazolidinyl urea, fatty alcohols such as cetearyl alcohol, cetyl alcohol and stearyl alcohol, pH adjusting agents such as citric acid, succinic acid, sodium hydroxide and triethanolamine, colouring agents such as any of the FD&C or D&C dyes, perfume oils, chelating agents such as ethylenediamine tetraacetic acid, and polymer plasticising agents such as glycerin and propylene glycol.

The invention also provides a method of styling hair by applying thereto a styling composition as is hereinabove described.

The following Examples further illustrate the preferred embodiments of the invention. All percentages referred to throughout this specification are by weight based on total weight unless otherwise indicated. Concentrations are based on 100% active ingredient.

### Examples

Cross-linked silicone was emulsion polymerised dimethiconol containing 0.6% crosslinker, 55% aqueous dispersion, ex Dow Corning.

Examples 1 to 8 illustrate hair styling compositions according to the invention.

The raw materials used in the examples include the following:

| Trade Name | Polymer Type | Supplier |
|---|---|---|
| Gafquat 755™ | Polyquaternium 11 | ISP |
| Gafquat 734™ | Polyquaternium 11 | ISP |
| Luviskol VA 64E™ | PVP/VA | BASF |

### Example 1

| | |
|---|---|
| 2.0% | Cross-linked silicone |
| 0.8% | PEG/VOH, PEG : vinyl acetate ratio 40:60, initial PEG molecular weight 35,000 Da, hydrolysed to vinyl alcohol to a level of more than 95%, cross-linked with 0.4 % by weight pentaerythritol triallyl ether |
| 0.8% | Polyquaternium 11 (added as Gafquat 755 ex ISP) |
| 0.3% | Volpo CS50 (ex Croda Oleochemicals) |
| 0.2% | Perfume |
| 8.0% | CAP40 (ex Calor Gas) |
| 0.05% | minors |
| water to 100% | |

### Example 2

| | |
|---|---|
| 2.0% | Cross-linked silicone |
| 1.0% | PEG/VOH, PEG : vinyl acetate ratio 40:60, initial PEG molecular weight 35,000 Da, hydrolysed to vinyl alcohol to a level of more than 95%, cross-linked with 0.4 % by weight pentaerythritol triallyl ether |
| 1.0% | Polyquaternium 11 (added as Gafquat 755 ex ISP) |
| 0.3% | Volpo CS50 (ex Croda Oleochemicals) |
| 0.2% | Perfume |
| 8.0% | CAP40 (ex Calor Gas) |
| 0.05% | minors |
| water to 100% | |

### Example 3

| | |
|---|---|
| 2.0% | Cross-linked silicone |
| 1.0% | PEG/VOH, PEG : vinyl acetate ratio 40:60, initial PEG molecular weight 35,000 Da, hydrolysed to vinyl alcohol to a level of more than 95%, cross-linked with 0.4 % by weight pentaerythritol triallyl ether |
| 0.8% | Polyquaternium 11 (added as Gafquat 755 ex ISP) |
| 0.3% | Volpo CS50 (ex Croda Oleochemicals) |
| 0.2% | Perfume |
| 8.0% | CAP40 (ex Calor Gas) |
| 0.05% | minors |
| water to 100% | |

### Example 4

| | |
|---|---|
| 2.0% | Cross-linked silicone |
| 4.0% | PEG/VOH, PEG : vinyl acetate ratio 40:60, initial PEG molecular weight 35,000 Da, hydrolysed to vinyl alcohol to a level of more than 95%, cross-linked with 0.4 % by weight pentaerythritol triallyl ether |
| 0.3% | Volpo CS50 (ex Croda Oleochemicals) |
| 0.2% | Perfume |
| 8.0% | CAP40 (ex Calor Gas) |
| 0.05% | minors |
| water to 100% | |

### Example 5

| | |
|---|---|
| 3.0% | Cross-linked silicone |
| 4.0% | PEG/VOH, PEG : vinyl acetate ratio 40:60, initial PEG molecular weight 35,000 Da, hydrolysed to vinyl alcohol to a level of more than 95%, cross-linked with 0.4 % by weight pentaerythritol triallyl ether |
| 1.5% | Polyquaternium 11 (added as Gafquat 755 ex ISP) |
| 0.3% | Volpo CS50 (ex Croda Oleochemicals) |
| 0.2% | Perfume |
| 8.0% | CAP40 (ex Calor Gas) |
| 0.05% | minors |
| water to 100% | |

### Example 6

| | |
|---|---|
| 2.5% | Cross-linked silicone |
| 2.0% | PEG/VOH, PEG : vinyl acetate ratio 40:60, initial PEG molecular weight 35,000 Da, hydrolysed to vinyl alcohol to a level of more than 95%, cross-linked with 0.4 % by weight pentaerythritol triallyl ether |
| 1.5% | Polyquaternium 11 (added as Gafquat 755 ex ISP) |
| 0.3% | Volpo CS50 (ex Croda Oleochemicals) |
| 0.2% | Perfume |
| 8.0% | CAP40 (ex Calor Gas) |
| 0.05% | minors |
| water to 100% | |

### Example 7

| | |
|---|---|
| 3.0% | Cross-linked silicone |
| 2.5% | PEG/VOH, PEG : vinyl acetate ratio 40:60, initial PEG molecular weight 35,000 Da, hydrolysed to vinyl alcohol to a level of more than 95%, cross-linked with 0.4 % by weight pentaerythritol triallyl ether |
| 2.0% | Polyquaternium 11 (added as Gafquat 755 ex ISP) |
| 0.3% | Volpo CS50 (ex Croda Oleochemicals) |
| 0.2% | Perfume |
| 8.0% | CAP40 (ex Calor Gas) |
| 0.05% | minors |
| water to 100% | |

### Example 8

| | |
|---|---|
| 2.0% | Cross-linked silicone |
| 3.0% | PEG/VOH, PEG : vinyl acetate ratio 40:60, initial PEG molecular weight 35,000 Da, hydrolysed to vinyl alcohol to a level of more than 95%, cross-linked with 0.4 % by weight pentaerythritol triallyl ether |
| 1.0% | Polyquaternium 11 (added as Gafquat 755 ex ISP) |
| 0.3% | Volpo CS50 (ex Croda Oleochemicals) |
| 0.2% | Perfume |
| 8.0% | CAP40 (ex Calor Gas) |
| 0.05% | minors |
| water to 100% | |

### Comparative Example 1

A composition was prepared according to Example 1 but containing 0.8% Gafquat 734 in place of the PEG/VOH copolymer.

### Comparative Example 2

A composition was prepared according to Example 1 but containing 4.0% PEG/VOH copolymer and no cross-linked silicone or polyquaternium polymer.

### Comparative Example 3

A composition was prepared according to Comparative Example 2 but containing 2.0% Gafquat 734 in place of the PEG/VOH copolymer.

### Comparative Example 4

A composition was prepared according to Comparative Example 2 but containing 4.0% Gafquat 734 in place of the PEG/VOH copolymer.

### Comparative Example 5

A composition was prepared according to Comparative Example 2 but containing 2.0% cross-linked silicone in place of the PEG/VOH copolymer.

### Comparative Example 6

A composition was prepared according to Comparative Example 2 but containing 3.0% cross-linked silicone and 3.0% Gafquat 734 in place of the PEG/VOH copolymer.

### Example 9

Compositions described above were tested by people using prototype products and scoring the compositions relative to each other.

| **Attribute** | Example 1 | Comp. Ex. 1 | Comp. Ex. 2 | Comp. Ex. 3 | Comp. Ex. 4 |
|---|---|---|---|---|---|
| Natural | 7.3 | 6.6 | 6.0 | 5.8 | 5.0 |
| Coated | 2.1 | 3.0 | 3.6 | 4.2 | 5.2 |
| Ease of Dry Comb | 9.4 | 9.5 | 8.8 | 8.4 | 6.9 |
| Hold | 7.2 | 7.3 | 7.0 | 7.0 | 6.9 |

This example shows the benefit of cross-linked silicone + PEG/VOH compositions compared to cross-linked silicone or the polymers used alone.

### Example 10

Compositions described above were tested by people using prototype products and scoring the compositions relative to each other.

| **Attribute** | Comp. Ex. 1 | Comp. Ex. 2 | Example 2 | Example 3 | Comp. Ex. 5 |
|---|---|---|---|---|---|
| Natural | 5.4 | 4.1 | 8.0 | 6.4 | 7.3 |
| Coated | 4.9 | 4.9 | 2.0 | 3.4 | 2.3 |
| Ease of Dry Comb | 9.0 | 7.8 | 10.0 | 9.0 | 8.9 |
| Hold | 7.9 | 5.7 | 8.6 | 7.7 | 7.7 |

This example shows the benefit of cross-linked silicone + PEG/VOH compositions compared to cross-linked silicone or polymers used alone, for different ratios of polymer to silicone, and cross-linker levels.

### Example 11

Compositions as described above were applied to curly switches of hair and the results assessed by experienced assessors.

| **Attribute** | Comp. Ex. 6 | Example 4 | Example 5 | Example 6 | Example 7 | Example 8 |
|---|---|---|---|---|---|---|
| Curl Definition | 5.0 | 5.5 | 6.5 | 7.5 | 6 | 7.0 |
| Soft Feel (dry) | 5.0 | 6.0 | 6.0 | 5.5 | 5.5 | 7.5 |
| Longevity | 5.0 | 5.0 | 5.0 | 5.0 | 6.2 | 5.3 |

This example shows the benefit of PEG/VOH containing products compared with cross-linked silicone products containing standard polymers.

## Claims

1. A hair styling composition comprising:
(i) from 0.1% to 10% by weight, based on total weight, of a non-rigid emulsion polymerised cross-linked silicone polymer, in which the percentage of branched monomer units in the silicone polymer is from 0.05% to 10%;
(ii) from 0.01% to 10% by weight, based on total weight, of a copolymer having a backbone comprising a polyether and, depending from polyethyleneglycol backbone, a plurality of poly(vinyl ester) groups, wherein at least 95% ester groups are hydrolysed to the corresponding alcohol and wherein the polymer has a molar ratio of polyether to total poly(vinyl ester) and polyvinyl alcohol groups from 30:70 to 50:50; and
(iii) water.

2. A hair styling composition according to claim 1, wherein the copolymer (ii) is polyethyleneglycol-co-polyvinylalcohol having polyvinylalcohol groups bound to the polyethyleneglycol.

3. A hair styling composition according to any one of the preceding claims, wherein the copolymer (ii) is obtainable by graft polymerisation.

4. A hair styling composition according to any one of the preceding claims, wherein the weight average molecular weight of the polyether is from 1 to 100 kDa.

5. A hair styling composition according to any one of the preceding claims, wherein the polymer (ii) is cross-linked.

6. A hair styling composition according to claim 5, wherein the cross-linking agent is pentaerythritol triallyl ether.

7. A hair styling composition according to any one of the preceding claims, further comprising from up to 10% by weight, based on total weight, of a hair styling polymer.

8. A hair styling composition according to claim 7, in which the hair styling polymer is an ionic-type hair styling polymer selected from Polyquaternium 11, Polyquaternium 16, Polyquaternium 46, and salts of monoalkyl esters of poly(methyl vinyl ether/maleic acid).

9. A hair styling composition according to any one of the preceding claims, in which the cross-linked silicone polymer (i) is a cross-linked dimethiconol, having a percentage of branched monomer units in the silicone polymer in the range 0.15% to 7%.

10. A hair styling composition according to any one of the preceding claims, which further comprising an alcohol selected from straight or branched chain monohydric alcohols having 2 to about 4 carbon atoms.

11. A hair styling composition according to any one of the preceding claims further comprising up to 5% by weight, based on total weight, of a non-ionic surfactant having an HLB value of at least 14.5.

12. A hair styling composition according to any one of the preceding claims, further comprising from 0% to 30% by weight, based on total weight, of an aerosol propellant.

13. A hair styling composition according to any one of the preceding claims which is an aerosol hair mousse in which the level of propellant is from 2% to 30% by weight, based on total weight.

14. A hair styling composition according to any one of claims 1 to 13, which is a hair styling cream or gel including from 0.01% to 10% by weight based on total weight of a structurant or thickener.

15. A method of styling hair which comprises applying to hair a composition of any one of claims 1 to 14.

16. The use of a composition of any one of claims 1 to 15 for the styling of hair.

## Patentansprüche

1. Haarformungszusammensetzung, die
(i) 0,1 bis 10 Gew.-%, bezogen auf das Gesamtgewicht, eines nicht-starren emulsionspolymerisierten quervernetzten Silikonpolymers, in welchem der Prozentanteil verzweigter Monomereinheiten im Silikonpolymer 0,05 % bis 10 % beträgt,
(ii) 0,01 bis 10 Gew.-%, bezogen auf das Gesamtgewicht, eines Copolymers mit einem Rückgrat, das ein Polyether und, in Abhängigkeit vom Polyethylenglycol-Rückgrat eine Mehrzahl von Poly(vinylester)gruppen umfasst, wobei mindestens 95 % der Estergruppen zum entsprechenden Alkohol hydrolysiert sind und wobei das Polymer ein molares Verhältnis von Polyether zu den gesamten Poly(vinylester)- und Polyvinylalkoholgruppen von 30:70 bis 50:50 aufweist, und
(iii) Wasser
umfasst.

2. Haarformungszusammensetzung nach Anspruch 1, wobei das Copolymer (ii) Polyethylenglycol-co-polyvinylalkohol, das mit dem Polyethylenglycol verbundene Polyvinylalkoholgruppen aufweist, ist.

3. Haarformungszusammensetzung nach einem der vorhergehenden Ansprüche, wobei das Copolymer (ii) durch Pfropfpolymerisation erhältlich ist.

4. Haarformungszusammensetzung nach einem der vorhergehenden Ansprüche, wobei das Gewichtsmittel des Molekulargewichts des Polyethers 1 bis 100 kDa beträgt.

5. Haarformungszusammensetzung nach einem der vorhergehenden Ansprüche, wobei das Polymer (ii) quervernetzt ist.

6. Haarformungszusammensetzung nach Anspruch 5, wobei das Quervernetzungsmittel Pentaerythritoltriallylether ist.

7. Haarformungszusammensetzung nach einem der vorhergehenden Ansprüche, die weiter bis zu 10 Gew.-%, bezogen auf das Gesamtgewicht, eines Haarformungspolymers umfasst.

8. Haarformungszusammensetzung nach Anspruch 7, in welcher das Haarformungspolymer ein Haarformungspolymer vom ionischen Typ ist, das aus Polyquaternium 11, Polyquaternium 16, Polyquaternium 46 und Salzen von Monoalkylestern von Poly(methylvinylether/maleinsäuze) ausgewählt ist.

9. Haarformungszusammensetzung nach einem der vorhergehenden Ansprüche, in welcher das quervernetzte Silikonpolymer (i) ein quervernetztes Dimethiconol mit einem Prozentanteil verzweigter Monomereinheiten im Silikonpolymer im Bereich von 0,15 % bis 7 % ist.

10. Haarformungszusammensetzung nach einem der vorhergehenden Ansprüche, die weiter einen Alkohol umfasst, der aus geradkettigen oder verzweigten einwertigen Alkoholen mit 2 bis etwa 4 Kohlenstoffatomen ausgewählt ist.

11. Haarformungszusammensetzung nach einem der vorhergehenden Ansprüche, die weiter bis zu 5 Gew.-%, bezogen auf das Gesamtgewicht, eines nicht-ionischen oberflächenaktiven Mittels mit einem HLB-Wert von mindestens 14,5 umfasst.

12. Haarformungszusammensetzung nach einem der vorhergehenden Ansprüche, die weiter 0 bis 30 Gew.-%, bezogen auf das Gesamtgewicht, eines Aerosoltreibmittels umfasst.

13. Haarformungszusarmensetzung nach einem der vorhergehenden Ansprüche, die ein Aerosolhaarschaum ist, in welchem der Gehalt des Treibmittels 2 bis 30 Gew.-%, bezogen auf das Gesamtgewicht, beträgt.

14. Haarformungszusammensetzung nach einem der Ansprüche 1 bis 13, die eine/ein Haarformungscreme oder -gel ist, die/das 0,01 bis 10 Gew.-%, bezogen auf das Gesamtgewicht, eines Strukturmittels oder Verdickungsmittels einschließt.

15. Verfahren zur Haarformung, welches das Auftragen einer Zusammensetzung nach einem der Ansprüche 1 bis 14 auf das Haar umfasst.

16. Verwendung einer Zusammensetzung nach einem der Ansprüche 1 bis 14 zur Haarformung.

## Revendications

1. Composition de coiffage comprenant :
(i) de 0,1% à 10% en poids, par rapport au poids total, d'un polymère de silicone souple, réticulé et polymérisé en émulsion, le pourcentage de motifs monomères ramifiés présents au sein du polymère de silicone valant de 0,05% à 10% ;
(ii) de 0,01% à 10% en poids, par rapport au poids total, d'un copolymère dont la chaîne principale comprend un polyéther et, selon la chaîne principale à base de polyéthylène glycol, une pluralité de groupes poly(ester vinylique), dans lequel au moins 95% des groupes ester sont hydrolysés en alcool correspondant et lequel polymère présente un rapport molaire du polyéther aux groupes poly(ester vinylique) et poly(alcool de vinyle) totaux qui vaut de 30/70 à 50/50 ; et
(iii) de l'eau.

2. Composition de coiffage selon la revendication 1, dans laquelle le copolymère (ii) représente un copolymère de polyéthylène glycol et de poly(alcool de vinyle) dans lequel les groupes poly(alcool de vinyle) sont liés au polyéthylène glycol.

3. Composition de coiffage selon l'une quelconque des revendications précédentes, dans laquelle le copolymère (ii) peut être obtenu par polymérisation par greffage.

4. Composition de coiffage selon l'une quelconque des revendications précédentes, dans laquelle la masse molaire moyenne en poids du polyéther vaut de 1 à 100 kDa.

5. Composition de coiffage selon l'une quelconque des revendications précédentes, dans laquelle le polymère (ii) est réticulé.

6. Composition de coiffage selon la revendication 5, dans laquelle l'agent de réticulation est l'éther triallylique de pentaérythritol.

7. Composition de coiffage selon l'une quelconque des revendications précédentes, comprenant en outre jusqu'à 10% en poids, par rapport au poids total, d'un polymère coiffant.

8. Composition de coiffage selon la revendication 7, dans laquelle le polymère coiffant est un polymère coiffant de type ionique choisi parmi le Polyquaternium 11, le Polyquaternium 16, le Polyquaternium 46, et des sels d'esters monoalkyliques du poly(éther méthylvinylique/acide maléique).

9. Composition de coiffage selon l'une quelconque des revendications précédentes, dans laquelle le polymère de silicone réticulé (i) représente un diméthiconol réticulé, le pourcentage de motifs monomères ramifiés au sein du polymère de silicone valant de 0,15% à 7%.

10. Composition de coiffage selon l'une quelconque des revendications précédentes, qui comprend en outre un alcool choisi parmi des alcools monohydriques à chaînes droites ou ramifiées qui comportent 2 à environ 4 atomes de carbone.

11. Composition de coiffage selon l'une quelconque des revendications précédentes, comprenant en outre jusqu'à 5% en poids, par rapport au poids total, d'un tensioactif non ionique dont la valeur HLB vaut au moins de 14,5.

12. Composition de coiffage selon l'une quelconque des revendications précédentes, comprenant en outre de 0% à 30% en poids, par rapport au poids total, d'un agent propulseur d'aérosols.

13. Composition de coiffage selon l'une quelconque des revendications précédentes, qui représente une mousse capillaire aérosol dans laquelle la proportion d'agent propulseur vaut de 2% à 30% en poids, par rapport au poids total.

14. Composition de coiffage selon l'une quelconque des revendications 1 à 13, qui représente un gel coiffant ou une crème de coiffage qui comprend de 0,01% à 10% en poids, par rapport au poids total, d'un agent structurant ou d'un épaississant.

15. Procédé de coiffage qui comprend l'application d'une composition selon l'une quelconque des revendications 1 à 14 sur les cheveux.

16. Utilisation d'une composition selon l'une quelconque des revendications 1 à 15, afin de coiffer les cheveux d'une certaine façon.
